# EUROPEAN PATENT SPECIFICATION

(11) **EP 1 803 456 B1**
(45) Date of publication and mention of the grant of the patent: **26.11.2014**
(21) Application number: 05708080.6
(22) Date of filing: 10.02.2005
(51) Int. Cl.: A61K 31/445, A61P 35/00

(54) **Pharmaceutical compositions comprising L-733060 for use in the treatment of cancer tumours**
Zusammensetzungen L-733060 enthaltend zur Verwendung bei der Behandlung von Krebstumoren
Compositions comprenant L-733060 pour leur utilisation dans le traitement des tumeurs cancéreuses

(30) Priority: 11.02.2004 ES 200400424
(43) Date of publication of application: 04.07.2007
(60) Divisional application: 14189674.6
(73) Proprietor: Munoz Sáez, Miguel, 41018 Sevilla (ES)
(72) Inventor: Munoz Sáez, Miguel, 41018 Sevilla (ES)
(74) Representative: Carpintero Lopez, Francisco
(86) International application number: PCT/ES2005/000068
(87) International publication number: WO 2005/077366

(56) References cited:
- EP-A2- 0 773 026
- EP-A2- 0 773 026
- WO-A1-98/54116
- WO-A1-98/54116
- WO-A2-01/01922
- WO-A2-01/01922
- MUNOZ M. ET AL: 'Antitumoral action of L-733.060 on neuroblastoma and glioma cell lines.' ARCHIVES ITALIENNES DE BIOLOGIE. vol. 142, no. 2, March 2004, pages 105 - 112, XP008079849
- MUNOZ M. ET AL: 'Antitumoral action of the neurokinin-1 receptor antaginist L-733060 on human malonoma cell lines.' MELANOMA RESEARCH. vol. 14, no. 3, June 2004, pages 183 - 188, XP008079852
- WEINSTEIN JOANNA L ET AL: "Advances in the diagnosis and treatment of neuroblastoma.", THE ONCOLOGIST 2003 LNKD- PUBMED:12773750, vol. 8, no. 3, 2003, pages 278-292, ISSN: 1083-7159
- MUNOZ M ET AL: "ANTITUMORAL ACTION OF THE NEUROKININ-1 RECEPTOR ANTAGINIST L-733060 ON HUMAN MALONOMA CELL LINES", MELANOMA RESEARCH, XX, XX, vol. 14, no. 3, 1 June 2004 (2004-06-01), pages 183-188, XP008079852,
- MIGUEL MUÑOZ ET AL: "NK-1 receptor antagonists induce apoptosis and counteract substance P-related mitogenesis in human laryngeal cancer cell line HEp-2", INVESTIGATIONAL NEW DRUGS ; THE JOURNAL OF NEW ANTICANCER AGENTS, KLUWER ACADEMIC PUBLISHERS, BO, vol. 26, no. 2, 29 September 2007 (2007-09-29), pages 111-118, XP019571876, ISSN: 1573-0646
- M. MUÑOZ: 'Antitumoral Action of the Neurokinin-1-Receptor Antagonist L-733,060 and Mitogenic Action of Substance P on Human Retinoblastoma Cell Lines' INVESTIGATIVE OPHTHALMOLOGY & VISUAL SCIENCE vol. 46, no. 7, 01 July 2005, pages 2567 - 2570, XP055005790 DOI: 10.1167/iovs.04-1530 ISSN: 0146-0404
- MUÑOZ M ET AL: 'ANTITUMORAL ACTION OF L-733.060 ON NEUROBLASTOMA AND GLIOMA CELL LINES' ARCHIVES ITALIENNES DE BIOLOGIE, PACINI, PISA, IT vol. 142, no. 2, 01 March 2004, pages 105 - 112, XP008079849 ISSN: 0003-9829
- MUÑOZ ET AL: 'A new frontier in the treatment of cancer: NK-1 receptor antagonists.' CURRENT MEDICINAL CHEMISTRY vol. 17, no. 6, 01 January 2010, pages 504 - 516, XP055005782 ISSN: 0929-8673
- MUÑOZ M ET AL: 'ANTITUMORAL ACTION OF THE NEUROKININ-1 RECEPTOR ANTAGINIST L-733060 ON HUMAN MALONOMA CELL LINES' MELANOMA RESEARCH vol. 14, no. 3, 01 June 2004, pages 183 - 188, XP008079852

## Description

### Object of the Invention

The object of the present invention is the use of substance P antagonists, particularly the use of non-peptide NK1 receptor antagonists for the treatment of cancer and more specifically on human melanoma, neuroblastoma, glioma, human Hodgkin's lymphoma, lymphoblastic leukemia, human breast carcinoma, human Ewing's sarcoma, human glioma and human osteosarcoma.

### State of the Art

Substance P is a natural undecapeptide belonging to the family of tachykinins, so called due to their rapid stimulant action on smooth muscle tissues. More specifically, substance P is a pharmacologically active neuropeptide which is produced in mammals (it was originally isolated in the intestine) and has an amino acid sequence which has been described by D. F. Veber et al. in US patent 4,680,283. The implication of substance P, as well as of other tachykinins, in the physiopathology in a large number of diseases has been widely demonstrated in the literature.

The substance P receptor is a member of the superfamily of G protein-coupled receptors. The neuropeptide substance P receptor (NK-1) is widely distributed in the nervous system of mammals (especially in the brain and in the spinal cord), the circulatory system and in peripheral tissues (especially in the duodenum and in the jejunum) and is involved in the regulation of various biological processes.

The central and peripheral action of tachykinin in mammals has been associated with several inflammatory conditions such as migraine, rheumatoid arthritis, asthma, and inflammatory bowel disease, as well as in the mediation of gag reflex and the regulation of central nervous (CNS) system disorders such as Parkinson's disease (Neurosci. Res., 1996, 7, 187-214), anxiety (Can. J. Phys.; 1997, 612-621) and depression (Science, 1998, 281, 1640-1645).

The review article entitled "Tachykinin Receptor and Tachykinin Receptor Antagonists", by J. Auton, in Pharmacol.; 1993, 13, 23-93, has described evidence on the usefulness of substance P antagonists for the treatment of headache, especially migraine, Alzheimer's disease, multiple sclerosis, attenuation of the opiate withdrawal syndrome, cardiovascular changes, edemas, such as edema caused by burns, chronic inflammatory diseases such as rheumatoid arthritis, asthma, bronchial hyperactivity, and other respiratory illnesses including allergic rhinitis, inflammatory bowel diseases, including ulcerative colitis and Crohn's disease, ocular lesions and ocular inflammation diseases.

Additionally, NK1 receptor antagonists are currently being developed as medicaments for the treatment of a large number of physiological disorders associated with an excess or an unbalance of tachykinins, particularly substance P. Thus, substance P has been related to nervous system disorders such as anxiety, depression and psychosis (WO 95/16679, WO 95/18124, WO 95/23798 and WO 01/77100).

NK1 receptor antagonists have additional usefulness for the treatment of motor diseases and vomiting induced by certain chemotherapy treatments.

The usefulness of NK1 receptor antagonists in the treatment of certain forms of urinary incontinence has been described in Neuropeptides, 32 (1), 1-49, 1998 and in Eur J. Pharmacol., 383(3), 297-303, 1999.

On the other hand, US 5972938 describes a method for the treatment of a psychoimmunological or psychomotor disorder by means of the administration of an NK1 receptor antagonist.

The article published in Nature, 2000, 405 (6783), 180-183 describes that mice lacking NK-1 receptors show a loss in the beneficial effect of morphine. Consequently, NK-1 receptor antagonists can be useful in the treatment of breaking the habit to certain addictive drugs such as opiates, nicotine as well as in the reduction of their abuse and withdrawal.

The article in Life Sci.; 2000, 67(9), 985-1001 describes that astrocytes express functional receptors for several neurotransmitters including substance P receptors. In brain tumors, malignant glial cells derived from astrocytes trigger, under the action of tachykinins by means of NK-1 receptors, the secretion of soluble mediators which increase the proliferation rate. Consequently, selective NK-1 antagonists can be very useful as therapeutic agents for the treatment of malignant gliomas and for the treatment of cancer.

Additionally, in the Never Journal of Medicine, 1999, 340, 190-195, it has been described that the use of a selective NK1 receptor antagonist induces the reduction of vomiting caused by the treatment with cisplatin.

The article published in the International Journal of Cancer by Antal Orosz et al. 1995, 60, 82-87, describes the use of various peptide substance P (SP) antagonists for inhibiting the proliferation of lung cell carcinoma (e.g., in NCl-H69 designated cells). In addition, the article published in Cancel Research, 1994, 54, 3602-3610, describes other also peptide SP antagonists capable of inhibiting the *in vitro* growth of several lung cancer cell lines (e.g., NCI-H510, NCI-H345 and SHP-77 designated cells).

Patent EP 773026 (Pfizer) describes the use of non-peptide NK1 receptor antagonists for the treatment of cancers in mammals, particularly in the treatment of small cell lung carcinomas in APUdoma, neuroendocrine tumors and extrapulmonary small cell carcinomas.

Additionally, patent WO 2001001922 describes the use of NK1 receptor antagonists for the treatment of adenocarcinoma and, very specifically, prostatic carcinomas. Giardina, G.; Gagliardi S. and Martinell M., "Antagonists at the neurokinin receptors - Recent patent literature (IDrugs 2005; 6(8): 758-772) review the most recent patents on NK1, NK2 and NK3 receptor antagonists. They describe the molecules of the most important worldwide manufacturers with a specific indication of the possible applications, among which it is important to point out: depression, Inflammation, anxiolytic, antiemetic, ulcerative colitis and others.

### Description of the Invention

The object of the present invention is the use of non-peptide NK1 receptor and substance P antagonists for the production of apoptosis in mammals tumor cells. The tumor cells on which the antagonists act have a much larger number of NK1 receptors than those existing in non-tumor cells, comprised between 400% and 500% of the number of usual receptors in non-tumor cells.

The tumor cells on which the antagonists act are selected from among:
- primary human invasive malignant melanoma cells
- metastatic human melanoma cells.
- melanoma cells located in lymph nodes
- human glioma cells
- human breast cancer cells
- human B cell acute lymphoblastic leukemia cells
- human T cell acute lymphoblastic leukemia cells
- human neuroblastoma-cells
- primary human neuroblastoma cells
- human astrocytoma cells
- human Hodgkin's lymphoma cells
- human Ewing's sarcoma cells
- human osteosarcoma cells.

The specific cell lines on which the non-peptide NK1 receptor and substance P antagonists act are indicated below. The tumor cells related to human melanoma on which the antagonists act belong to the cell lines COLO 858 [ICLC, Interlab Cell Line Collection -CBA-Genoa), MEL HO [DSMZ, Deutsche Sammlung von Mikroorganismen und Zellkulturen] and COLO 679 [DSMZ].

The tumor cells related to human glioma and human neuroblastoma on which the antagonists act belong to the cell lines GAMG [DSMZ] and SKN-BE (2) [ICLC].

The tumor cells related to lymphoblastic leukemia on which the antagonists act belong to the cell lines of human B cell lymphoblastic leukemia SD1 [DSMZ] and human T cell lymphoblastic leukemia BE-13 [DSMZ].

The tumor cells related to human Hodgkin's lymphoma on which the antagonists act belong to the cell line KM-H2 [DSMZ].

The tumor cells related to human breast cancer on which the antagonists act belong to the cell line MT-3 [DSMZ].

The tumor cells related to human Ewing's sarcoma on which the antagonists act belong to the cell line MHH-ES-1 [DSMZ].

The tumor cells related to human osteosarcoma on which the antagonists act belong to the cell line MG-63 [ICLC].

The antagonist used in the present invention is (2S,3S)3-{[3,5-Bis(trifluoromethyl)phenyl]methoxy)-2-phenylpiperidine, commercially known as L-733060 (Sigma-Aldrich) and it has been used at concentrations comprised between 5 µM and 50 µM.

Other non-peptide NK1 receptor and substance P antagonist compounds described herein are:
- vofopitant or GR-205171 (Pfizer),
- ezlopitant or CJ-11974 (Pfizer),
- CP-122721 (Pfizer),
- aprepitant or MK 869 or L-754030 (MSD),
- L-758298 (MSD),
- TAK-637 (Takeda/Abbot),
- GW597599 (GSK),
- GW679769 (GSK),
- R673 (Roche).

Finally, another object of the present invention is the use of the non-peptide NK1 receptor and substance P antagonist as claimed for the preparation of a pharmaceutical composition for the treatment of cancer.

### Brief Description of the Drawings

Figures 1A and 1B: variation over time of the concentrations of SKN-BE(2) cells at increasing concentrations of L-733,060 (1A) and cell growth inhibition of SKN-BE(2) (1 B).
Figures 2A and 2B: variation over time of the concentration of COLO 858 cells at increasing concentrations of L-733,060 (2A) and cell growth inhibition of COLO 858 (2B).
Figures 3A and 3B: variation over time of the concentration of MEL HO cells at increasing concentrations of L-733,060 (3A) and cell growth inhibition of MEL HO (3B).
Figures 4A and 4B: variation over time of the concentration of COLO 679 cells at increasing concentrations of L-733,060 (4A) and cell growth inhibition of COLO 679 (48).
Figures 5A and 5B: variation over time of the concentration of SD1 cells at increasing concentrations of L-733,060 (5A) and cell growth inhibition of SD1 (5B).
Figures 6A and 6B: variation over time of the concentration of KM-H2 cells at increasing concentrations of L-733,060 (6A) and cell growth inhibition of KM-H2 (6B).
Figures 7A and 7B: variation over time of the concentration of MT-3 cells at increasing concentrations of L-733,060 (7A) and cell growth inhibition of MT-3 (7B).
Figures 8A and 8B: variation over time of the concentration of MHH-ES-1 cells at increasing concentrations of L-733,060 (8A) and cell growth inhibition of MHH-ES-1 (8B).
Figures 9A and 9B: variation over time of the concentration of MG-63 cells at increasing concentrations of L-733,060 (9A) and cell growth inhibition of MG-63 (9B).
Figures 10A and 10B: variation over time of the concentration of GAMG cells at increasing concentrations of L-733,060 (10A) and cell growth inhibition of GAMG(10B).

### Detailed Description of the Invention

A detailed explanation of how the experiments on which the present invention is based were conducted for each tested cell line is included below.

### Example 1: Cell lines related to neuroblastoma:

The human neuroblastoma cell line SKN-BE (2) (ICLC Interlab Cell Line Collection-CBA-Genoa) was used.

This line was maintained in RPMI 1640 culture medium (GIBCO-BRL) supplemented with 10% fetal bovine serum according to the cell culture conditions of the ATCC.

The cell line was cultured in 75 ml flasks (Falcon, Germany). The medium was refreshed every two days and the cells were treated with trypsin (0.05% and 0.02% EDTA without Ca²+ and Mg²+) every six days. The cells were incubated at 37ºC under humidification (95% air/5% CO2).

### Treatment with the NK1 receptor antagonist:

The solutions of the NK1 receptor antagonist (2S,3S)3-([3,5Bis (trifluoromethyl)phenyl]methoxy)-2-phenylpiperidine (L-733,060) (Sigma-Atdrich, U.K.), were dissolved in distilled water containing 0.2% dimethyl sulfoxide (DMSO) before treating the samples. Different concentrations (2.5 µM to 20 µM) were studied for the purpose of determining the IC₅₀.

Cell proliferation was evaluated using the MTS [3-(4,5-dimethylthiazol-2-yl)-5-(3-carboxymethoxyphenyl)2-(4-sulfophenyl)-2H-tetrazolium] method, following the instructions for use (CellTiter 96 Aqueous One Solution Cell Proliferation Assay, Promega, USA).

### Cell proliferation method

During the time of the experiment the cultured cells were detached every 4-5 days by means of trypsinization and the trypan blue method was used for cell viability. The cells were quantified by means of a counting method and cultured in 96-well plates. Each experiment included three plates termed To, T₁ and T₂.

To contained wells without cells (0 cells/0.1 ml) termed white wells and wells that contained cells (10⁴ cells/0.1 ml) were termed control wells. Both T₁ and T₂ included white wells (0 cells/0.1 ml), control wells (10⁴ cells/0.1 ml) and control wells treated with L-733,060.

In To, 20 µl of MTS were immediately added to the wells and the wells were read 90 minutes later. T₁ and T₂ were treated with different concentrations (2.5 µM to 20 µM) of L-733,060 and were incubated during a period of 30 hours (first cell division) (T₁) and 72 hours (second cell division) (T₂).

To study cell proliferation, 20 µl of MTS reagent were added to each well (T₁,T₂) 90 minutes before reading the samples with the plate reader (TECAN. Spectra Classic) at 492 nm. The amount of MTS reagent was measured by optical density being directly proportional to the number of live cells. Each plate (white, control, and control treated with different concentrations of L-733,060) was done in triplicate. The experiment was repeated on three different occasions. The concentration to inhibit fifty percent of the cells (IC₅₀) with L-733,060 was calculated with a curve suited to the parameters.

### Statistical analysis

The data obtained was evaluated using the student's t test, with a level of significance of P< 0.05.

### Results

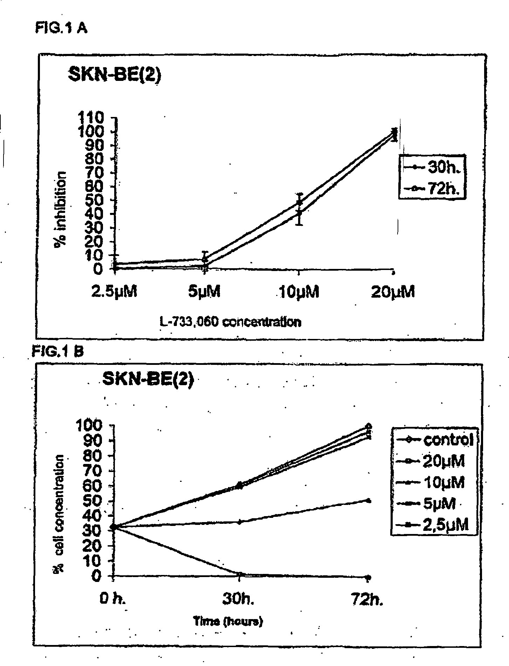

The results shown in Figure 1A represent the variation over time of the concentration of SKN-BE (2) cells at increasing concentrations of L-733,060.

Figure 1B shows cell growth inhibition of SKN-BE (2) (at 30 hours and 72 hours) after the addition of increasing concentrations of L-733,060 (2.5, 5, 10, 20 µM) and the percentage of inhibition for the first and second time.of division of the incubation. The non-continuous lines represent IC₅₀ for 30 and 72 hours. The points on the graph represent the mean value ± standard deviation.

### Example 2: Cell lines related to melanomas

Human melanoma cell lines COLO 858 (ICLC Interlab Cell Line Collection-CBA-Genoa), MEL HO and COLO 679 (DSMZ- Deutsche Sammlung von Mikroorganismen und Zellkulturen) were used.

This cell line was maintained: in 1640 RPMI culture medium (GIBCO-BRL) supplemented with 10% fetal bovine serum according to the cell culture conditions of the ATCC, the ICLC and the DSMZ.

The cell lines were cultured in 75 ml flasks (Falcon, Germany). The medium was refreshed every two days and the cells were treated with trypsin (0.05% and 0.02% EDTA without Ca²+ and Mg²+) every six days. The cells were incubated at 37ºC under humidification (95% air/5% CO2).

### Treatment with the NK1 receptor antagonist

The solutions of the NK1 receptor antagonist (2S,3S)3-([3,5Bis(trifluoromethyl)phenyl]methoxy)-2-phenylpiperidine (L-733,060) (Sigma-Aldrich, U.K.) were dissolved in distilled water containing 0.2% dimethyl sulfoxide (DMSO) before treating the samples. Different concentrations (2.5 µM to 50 µM) were studied for the purpose of determining the IC₅₀.

Cell proliferation was evaluated using the MTS [3-(4,5-dimethylthiazol-2-yl)-5-(3-carboxymethoxyphenyl)2-(4-sulfophenyl)-2H-tetrazolium] method, following the instructions for use (CellTiter 96 Aqueous One Solution Cell Proliferation Assay, Promega, USA).

### Cell proliferation method

During the time of the experiment, the cultured cells were detached every 4-5 days by means of trypsinization and the trypan blue method was used for cell viability. The cells were quantified by means of a counting method and cultured in 96-well plates. Each experiment included three plates termed T₀, T₁ and T₂.

To contained wells without cells (0 cells/0.1 ml) termed white wells and wells that contained cells (10⁴ cells/0.1 ml) were termed control wells. Both, T₁ and T₂ 2 included white wells (0 cells/0.1 ml), control wells (10⁴ cells/0.1 ml) and control wells treated with L-733,060.

In T₀, 20 µl of MTS were immediately added to the wells and they were read 90 minutes later. T₁ and T₂ were treated with different concentrations (2.5 µM to 50 µM) of L-733,060 and were incubated for a variable period for each cell line.

Line COLO 858: 48 hours (first cell division) (T₁) and 96 hours (second cell division) (T₂).

Line MEL HO: 24 hours (first cell division) (T₁) and 48 hours (second cell division) (T₂).

Line COLO 679: 30 hours (first cell division) (T₁) and 72 hours (second cell division) (T₂).

To study cell proliferation, 20 µl of MTS reagent were added to each well (T₁, T₂) 90 minutes before reading the samples with the plate reader (TECAN Spectra Classic) at 492 nm. The amount of MTS reagent was measured by optical density, being directly proportional to the number of live cells. Each plate (white, control, and control treated with different concentrations of L-733,060) was done in triplicate. The experiment was repeated on three different occasions. The concentrations to inhibit fifty percent of the cells (IC₅₀) with L-733,060 was calculated with a curve suited to the parameters.

### Statistical analysis

The data obtained was evaluated using the student's t test, with a level of significance of P< 0.05.

### Results

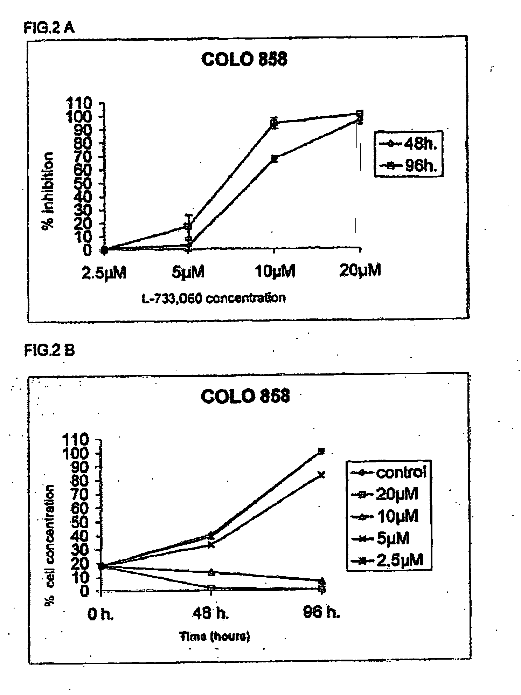

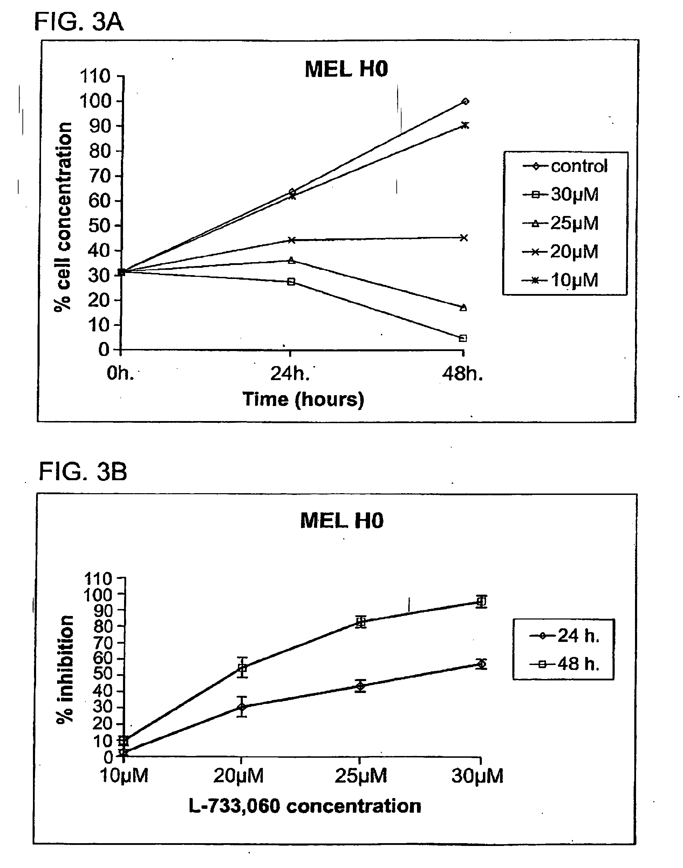

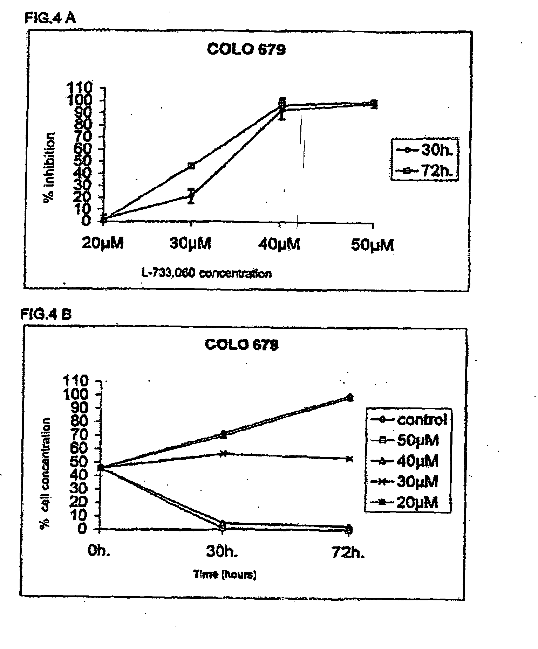

The results are shown in Figures 2A and 2B (COLO 858), Figures 3A and 3B (MEL HO) and Figures 4A and 4B (COLO 679).

Figure 2A represents the variation over time of the concentration of COLO 858 cells at increasing concentrations of L-733,060 (from 2.5 to 20 µM).

Figure 3A represents the variation over time of the concentration of MEL HO cells at increasing concentrations of L-733,060 (from 10 to 30 µM).

Figure 4A represents the variation over time of the concentration of COLO 679 cells at increasing concentrations of L-733,060 (from 20 to 50 µM).

Figure 2B shows cell growth inhibition of COLO 858 (at 48 and 96 hours) after the addition of increasing concentrations of L-733,060 (2.5, 5, 10, 20 µM). The discontinuous lines represent the IC₅₀ for 48 and 96 hours. The points on the graph represent the mean value ± standard deviation.

Figure 3B shows cell growth inhibition of MEL HO (at 24 and 48 hours) after the addition of increasing concentrations of L-733,060 (10, 20, 25 and 30 µM). The discontinuous lines represent the IC₅₀ for 24 and 48 hours. The points on the graph represent the mean value ± standard deviation.

Figure 4B shows cell growth inhibition of COLO 679 (at 30 and 72 hours after the addition of increasing concentrations of L-733,060 (20, 30, 40 and 50 µM). The discontinuous lines represent the IC₅₀ for 30 and 72 hours. The points on the graph represent the mean value ± standard deviation.

### Example 3: Cell lines related to lymphoblastic leukemia.

Human lymphoblastic leukemia cell lines of B SD1 (DSMZ) cells and T BE-13 (DSMZ) cells were used.

These cell lines were maintained in 1640 RPMI culture medium supplemented with 10% fetal bovine serum according to the cell culture conditions of the ATCC.

The cell line was cultured in 75 ml flasks (Falcon, Germany). The medium was refreshed every two days. The cells were incubated at 37ºC under humidification (95% air/5% CO2).

### Treatment with the NK1 receptor antagonist

The solutions of the NK1 receptor antagonist (2S,3S)3-([3,5-Bis (trifluoromethyl)phenyl]methoxy)-2-phenylpiperidine (L-733,060) (Sigma-Aldrich, U.K.) were dissolved in distilled water containing 0.2% dimethyl sulfoxide (DMSO) before treating the samples. Different concentrations (2.5 µm to 25 µM) were studied for the purpose of determining the IC₅₀.

Cell proliferation was evaluated using the MTS [3-(4,5-dimethylthiazol-2-yl)-5-(3-carboxymethoxyphenyl)2-(4-sulfophenyl)-2H-tetrazolium] method, following the instructions for use (CellTiter 96 Aqueous One Solution Cell Proliferation Assay, Promega, USA).

### Cell proliferation, method

The cells were quantified by means of a counting method and cultured in 96-well plates. Each experiment included three plates termed T₀, T₁ and T₂.

T₀ contained wells without cells (0 cells/0.1 ml) termed white wells and wells that contained cells (10⁴ cells/0.1 ml) were termed control wells. Both, T₁ and T₂ ,included white wells (0 cells/0.1 ml), control wells (10⁴ cells/0.1 ml) and control wells treated with L-733,060.

In T₀, 20 µl of MTS were immediately added to the wells and they were read 90 minutes later. T₁ and T₂ were treated with different concentrations (2.5 µM to 20 µM) of L-733,060 and were incubated during a period of 30 hours (first cell division) (T₁) and 72 hours (second cell division)(T₂).

To study cell proliferation, 20 µl of MTS reagent were added to each well (T₁, T₂) 180 minutes before reading the samples with the plate reader (TECAN Spectra Classic) at 492 nm. The amount of MTS reagent was measured by optical density, being directly proportional to the number of live cells. Each plate (white, control, and control treated with different concentrations of L-733,060) was done in triplicate. The experiment was repeated on three different occasions. The concentration to inhibit fifty percent of the cells (IC₅₀) with L-733,060 was calculated with a curve suited to the parameters.

### Statistical analysis

The data obtained was evaluated using the student's t test, with a level of significance of P< 0.05.

### Results

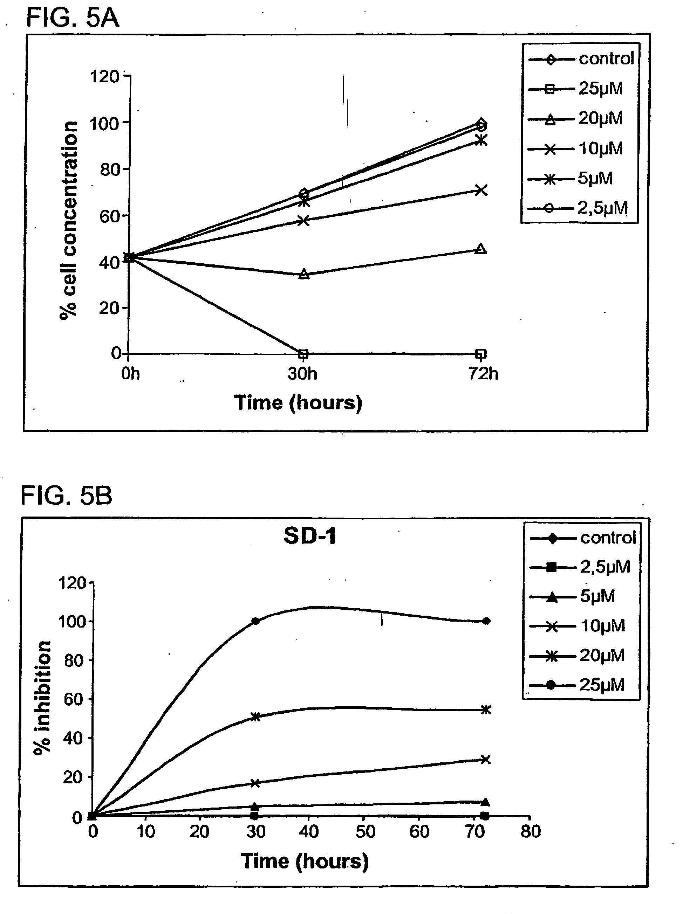

The results shown in Figure 5A represent the variation over time of the concentration of B SD1 cells at increasing concentrations of L-733,060.

Figure 5B shows cell growth inhibition of B SD1 (at 30 and 72 hours) after the addition of increasing concentrations of L-733,060 (2.5, 5, 10, 25 µM) and the percentage of inhibition for the first and second time of division of the incubation. The discontinuous lines represent the IC₅₀ for 30 and. 72 hours The points on the graph represent the mean value ± standard deviation.

### Example 4: Cell lines related to Burkitt's human lymphoma. (not part of the invention)

Human Burkitt's lymphoma cell line CA-46 (DSMZ) was used.

This cell line was maintained in 1640 RPMI culture medium supplemented with 10% fetal bovine serum according to the cell culture conditions of the ATCC.

The cell line was cultured in 75 ml flasks (Falcon, Germany). The medium was refreshed every two days and the cells were treated with trypsin (0.05% and 0.02% EDTA without Ca²+ and Mg²+) every six days. The cells were incubated at 37ºC under humidification (95% air/5% CO2).

### Treatment with the NK1 receptor antagonist

The solutions of the NK1 receptor antagonist (2S,3S)3-([3,5-Bis(trifluoromethyl)phenyl]methoxy)-2-phenylpiperidine (L-733,060) (Sigma-Aldrich, U.K.) were dissolved in distilled water containing 0.2% dimethyl sulfoxide (DMSO) before treating the samples. Different concentrations (2.5 µM to 25 µM) were studied for the purpose of determining the IC₅₀.

Cell proliferation was evaluated using the MTS [3-(4,5-dimethylthiazol-2-yl)-5-(3-carboxymethoxyphenyl)2-(4-sulfophenyl)-2H-tetrazolium] method, following the instructions for use (CellTiter 96 Aqueous One Solution Cell Proliferation Assay, Promega, USA).

### Cell proliferation method

The cells were quantified by means of a counting method and cultured in 96-well plates. Each experiment.included three plates term.ed To, T₁ and T₂.

T₀ contained wells without cells (0 cells/0.1 ml) termed white wells and wells that contained cells (10⁴ cells/0.1 ml) were termed control wells. Both T₁ and T₂ included white wells (0 cells/0.1 ml), control wells (10⁴ cells/0.1 ml) and control wells treated with L-733,060.

In To, 20 µl of MTS were immediately added to the wells and they were read 90 minutes later. T₁ and T₂ were treated with different concentrations (2.5 µM to 25 µM) of L-733,060 and were incubated during a period of 35 hours (first cell division) (T₁) and 72 hours (second cell division)(T₂).

To study cell proliferation, 20 µl of MTS reagent were added to each well (T₁, T₂) 90 minutes before reading the samples with the plate reader (TECAN Spectra Classic) at 492 nm. The amount of MTS reagent was measured by optical density, being directly proportional to the number of live cells. Each plate (white, control, and control treated with different concentrations of L-733,060) was done in triplicate. The experiment was repeated on three different occasions. The concentration to inhibit fifty percent of the cells (IC₅₀) with L-733,060 was calculated with a curve suited to the parameters.

### Statistical analysis

The data obtained was evaluated using the student's t test, with a level of significance of P<0.05.

### Results

Cell growth inhibition occurred at the highest concentrations and apoptosis occurred at the maximum dose.

### Example 5: Cell lines related to human Hodgkin's lymphoma

Human Hodgkin's lymphoma cell line KM-H2 (DSMZ) was used.

This cell line was maintained in 1640 RPMI culture medium supplemented with 10% fetal bovine serum according to the established cell culture conditions of the ATCC.

The cell line was cultured in 75 ml flasks (Falcon, Germany). The medium was refreshed every two days. The cells, were incubated at 37°C under humidification (95% air/5% CO2):

### Treatment with the NK1 receptor antagonist

The solutions of the NK1 receptor antagonist (2S,3S)3-([3,5-Bis(trifluoromethyl)phenyl]methoxy)-2-phenylpiperidine (L-733,060) (Sigma-Aldrich, U.K.) were dissolved in distilled water containing 0.2% dimethyl sulfoxide (DMSO) before treating the samples. Different concentrations (2.5 µM to 25 µM) were studied for the purpose of determining the IC₅₀.

Cell proliferation was evaluated using the MTS [3-(4,5- dimethylthiazol-2-yl)-5-(3-carboxymethoxyphenyl)2-(4-sulfophenyl)-2H-tetrazolium] method; following the instructions for use (CellTiter 96 Aqueous One Solution Cell Proliferation Assay, Promega, USA).

### Cell proliferation method

The cells were quantified by means of a counting method and cultured in 96-well plates. Each experiment included three plates termed T₀, T₁ and T₂.

T₀ contained wells without cells (0 cells/0.1 ml) termed white wells and wells that contained cells (10⁴ cells/0.1 ml) were termed control wells. Both T₁ and T₂ included white wells (0 cells/0.1 ml), control wells (10⁴ cells/0.1 ml) and control wells treated with L-733,060.

In T₀, 20 µl of MTS were immediately added to the wells and they were read 90 minutes later. T₁ and T₂ were treated with different concentrations (2.5 µM to 20 µM) of L-733,060 and were incubated during a period of 48 hours (first cell division) (T1) and 96 hours (second cell division) (T₂).

To study cell proliferation, 20 µl of MTS reagent were added to each well (T₁, T₂) 180 minutes before reading the samples with the plate reader (TECAN Spectra Classic) at 492 nm. The amount of MTS reagent was measured by optical density, bering directly proportional to the number of live cells. Each plate (white, control, and control treated with different concentrations of L-733,060) was done in triplicate. The experiment was repeated on three different occasions. The concentration to inhibit fifty percent of the cells (IC₅₀) with L-733,060 was calculated with a curve suited to the parameters.

### Statistical analysis

The data obtained was evaluated using the student's t test, with a level of significance of P< 0.05.

### Results

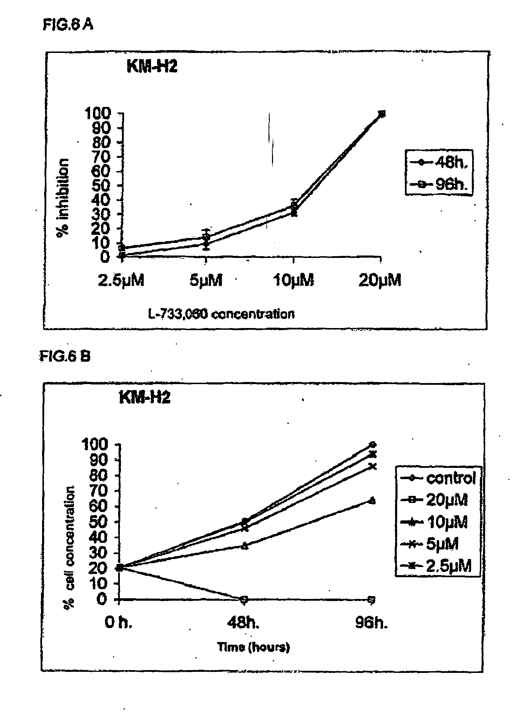

The results shown in Figure 6A represent the variation over time of the concentration of KM-H2 cells with increasing concentrations of L-733,060.

Figure 6B shows cell growth inhibition of KM-H2 (at 48 and 96 hours) after the addition of increasing concentrations of L-733,060 (2.5, 5, 10, 20µM) and the percentage of inhibition for the first and second time of division of the incubation. The discontinuous lines represent the IC₅₀ for 48 and 96 hours. The points on the graph represent the mean value ± standard deviation.

### Example 6: Cell lines related to human rhabdomyosarcoma. (not part of the invention)

Human rhabdomyosarcoma cell line A-204 (DSMZ) was used.

This cell line was maintained in McCoy's culture medium supplemented with 10% fetal bovine serum according to the cell culture conditions of the ATCC.

The cell line was cultured in 75 ml flasks (Falcon, Germany). The medium was refreshed every two days and the cells were treated with trypsin (0.05% and 0.02% EDTA without Ca²+ and Mg²+) every six days. The cells were incubated at 37ºC under humidification (95% air/5% CO2).

### Treatment with the NK1 receptor antagonist

The solutions of the NK1 receptor antagonist (2S,3S)3-([3,5-Bis(trifluoromethyl)phenyl]methoxy)-2-phenylpiperidine (L=733,060) (Sigma-Aldrich, U.K.) were dissolved in distilled water containing 0.2% dimethyl sulfoxide (DMSO) before treating the samples. Different concentrations (2.5 µM to 25µM) were studied for the purpose of determining the IC₅₀.

Cell proliferation was evaluated using the MTS [3-(4,5-dimethylthiazol-2-yl)-5-(3-carboxymethoxyphenyl)2-(4-sulfophenyl)-2H-tetrazolium] method, following the instructions for use (Cell Titer 96 Aqueous One Solution Cell Proliferation Assay, Promega, USA).

### Cell proliferation method

During the time of the experiment, the cultured cells were detached every 4-5 days by means of trypsinization and the trypan blue method was used for cell viability. The cells were quantified by means of a counting method and cultured in 96-well plates. Each experiment included three plates termed T₀, T₁ and T2.

T₀ contained wells without cells (0 cells/0.1 ml) termed white wells and wells that contained cells (10⁴ cells/0.1 ml) were termed control wells. Both T₁ and T₂ included white wells (0 cells/0.1 ml), control wells (10⁴ cells/0.1 ml) and control wells treated with L-733,060.

In T₀, 20 µl of MTS were immediately added to the wells and they were read 90 minutes later. T₁ and T₂ were treated with different concentrations (5 µM to 25 µM) of L-733,060 and were incubated during a period of 36 hours (first cell division) (T₁) and 72 hours (second cell division) (T₂).

To study cell proliferation, 20 µl of MTS reagent were added to each well (T₁, T₂) 90 minutes before reading the samples with the plate reader (TECAN Spectra Classic) at 492 nm. The amount of MTS reagent was measured by optical density, being directly proportional to the number of live cells. Each plate (white, control, and control treated with different concentrations of L-733,060) was done in triplicate. The experiment was repeated on three different occasions. The concentration to inhibit fifty percent of the cells (IC₅₀) with L-733,060 was calculated with a curve suited to the parameters.

### Statistical analysis

The data obtained was evaluated using the student's t test, with a level of significance of P< 0.05.

### Results

Cell growth inhibition occurred at the highest concentrations and apoptosis occurred at the maximum dose.

### Example 7: Cell lines related to small cell lung carcinoma (not part of the invention)

Small cell lung carcinoma cell line COLO-677 (DSMZ) was used. This cell line was maintained in 1640 RPMI culture medium supplemented with 10% fetal bovine serum according to the cell culture conditions of the ATCC.

The cell line was cultured in 75 ml flasks (Falcon, Germany). The medium was refreshed every two days and the cells were treated with trypsin (0.05% and 0.02% EDTA without Ca²+ and Mg²+) every six days. The cells were incubated at 37ºC under humidification (95% air/5% CO2).

### Treatment with the NK1 receptor antagonist

The solutions of the NK1 receptor antagonist (2S,3S)3-([3,5-Bis(trifluoromethyl)phenyl]methoxy)-2-phenylpiperidine (L-733,060) (Sigma-Aldrich, U.K.) were dissolved in distilled water containing 0.2% dimethyl sulfoxide (DMSO) before treating the samples. Different concentrations (2.5 µM to 25 µM) were studied for the purpose of determining.the IC₅₀.

Cell proliferation was evaluated using the MTS [3-(4,5-dimethylthiazol-2-yl)-5-(3-carboxymethoxyphenyl)2-(4-sulfophenyl)-2H-tetrazolium] method, following the instructions for use (CellTiter 96 Aqueous One Solution Cell Proliferation Assay, Promega, USA).

### Cell proliferation method

During the time of the experiment, the cultured cells were detached every 4-5 days by means of trypsinization and the trypan blue method was used for cell viability. The cells were quantified by means of a counting method and cultured in 96-well plates. Each experiment included three plates termed To, T₁ and T₂.

T₀ contained wells without cells (0 cells/0.1 ml) termed white wells and wells that contained cells (10⁴ cells/0.1 ml) were termed control wells.. Both T₁ and T₂ included white wells (0 cells/0.1 ml), control wells, (10⁴ cells/0.1 ml) and control wells treated with L-733,060.

In T₀, 20 µl of MTS were immediately added to the wells and they were read 90 minutes later. T₁ and T₂ were treated with different concentrations (5 µM to 20 µM) of L-733,060 and were incubated during a period of 40 hours (first cell division) (T₁) and 96 hours (second cell division) (T₂).

To study cell proliferation, 20 µl of MTS reagent were added to each well (T₁,T₂) 90 minutes before reading the samples with the plate reader (TECAN Spectra Classic) at 492 nm. The amount of MTS reagent was measured by optical density, being directly proportional to the number of live cells. Each plate (white, control, and control treated with different concentrations of L-733,060) was done in triplicate. The experiment was repeated on three different occasions. The concentration to inhibit fifty percent of the cells (IC₅₀) with L-733,060 was calculated with a curve suited to the parameters.

### Statistical analysis.

The data obtained was evaluated using the student's t test, with a level of significance of P< 0.05.

### Results

Cell growth inhibition occurred at the highest concentrations and apoptosis occurred at the maximum dose.

### Example 8: Cell lines related to human breast cancer

Human breast cancer cell line MT-3 (DSMZ) was used. This cell line was maintained in 1640 RPMI culture medium supplemented with 10% fetal bovine serum according to the cell culture conditions of the ATCC.

The cell line was cultured in 75 ml flasks (Falcon, Germany). The medium was refreshed every two days and the cells were treated with trypsin (0.05% and 0.02% EDTA without Ca²+ and Mg²+) every six days. The cells were incubated at 37ºC under humidification (95% air/5% CO2).

### Treatment with the NK1 receptor antagonist

the solutions of the NK1 receptor antagonist (2S,3S)3-([3,5-Bis(trifluoromethyl)phenyl]methoxy)-2-phenylpiperidine (L-733,060) (Sigma-Aldrich, U.K.) were dissolved in distilled water containing 0.2% dimethyl sulfoxide (DMSO)before treating the samples. Different concentrations (2.5 µM to 25 µM) were studied for the purpose of determining the IC₅₀.

Cell proliferation was evaluated using the MTS [3-(4,5-dimethylthiazol-2-yl)-5-(3-carboxymethoxyphenyl)2-(4-sulfophenyl)-2H-tetrazolium] method, following the instructions for use (CellTiter 96 Aqueous One Solution Cell Proliferation Assay, Promega, USA).

### Cell proliferation method

During the time of the experiment, the cultured cells were detached every 4-5 days by means of trypsinization and the trypan blue method was used for cell viability. The cells were quantified by means of a counting method and cultured in 96-well plates. Each experiment included three plates termed T₀, T₁ and T₂.

To contained wells without cells (0 cells/0.1 ml) termed white wells and wells that contained cells (10⁴ cells/0.1 ml) were termed control wells. Both T₁ and T₂ included white wells (0 cells/0.1 ml), control wells (10⁴ cells/0.1 ml) and control wells treated with L-733,060.

In T₀, 20 µl of MTS were immediately added to the wells and they were read 90 minutes later. T₁ and T₂ were treated with different concentrations (2.5 µM to 20 µM) of L-733,060 and were incubated during a period of 30 hours (first cell division) (T₁) and 72 hours (second cell division) (T₂).

To study cell proliferation, 20 µl of MTS reagent were added to each well (T₁, T₂) 90 minutes before reading the samples with the plate reader (TECAN Spectra Classic) at 492 nm. The amount of MTS reagent was measured by optical density, being directly proportional to the number of live cells. Each plate (white, control, and control treated with different concentrations of L-733,06.0) was done in triplicate. The experiment was repeated on three different occasions. The concentration to inhibit fifty percent of the cells (IC₅₀) with L-733,060 was calculated with a curve suited to the parameters.

### Statistical analysis

The data obtained was evaluated using the student's t test, with a level of significance of P< 0.05.

### Results

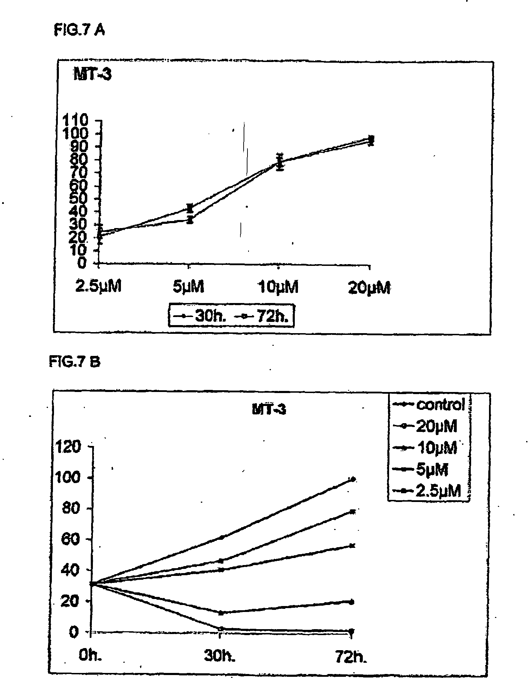

The results shown in Figure 7A represent the variation over time of the concentration of MT-3 cells at increasing concentrations of L-733,060.

Figure 7B shows cell growth inhibition of MT-3 (at 30 and 72 hours) after the addition of increasing concentrations of L-733,060 (2.5, 5, 10, 20 µM) and the percentage of inhibition for the first and second time of division of the incubation. The discontinuous lines represent the IC₅₀ for 30 and 72 hours. The points on the graph represent the mean value ± standard deviation.

### Example 9: Cell lines related to Ewing's human sarcoma

Ewing's human sarcoma cell line MHH-ES-1 (DSMZ) was used. This cell line was maintained in 1640 RPMI culture medium supplemented with 10% fetal bovine serum according to the cell culture conditions of the ATCC.

The cell line was cultured in 75 ml flasks (Falcon, Germany), The medium was refreshed every two days and the cells were treated with trypsin (0.05% and 0.02% EDTA without Ca²+ and Mg²+) every six days. The cells were incubated at 37ºC under humidification (95% air/5% CO2).

### Treatment with the NK1 receptor antagonist

The solutions of the NK1 receptor antagonist (2S,3S)3-([3,5-Bis(trifluoromethyl)phenyl]methoxy)-2-phenylpiperidine (L-733,060) (Sigma-Aldrich, U.K.) were dissolved in distilled water containing 0.2% dimethyl sulfoxide (DMSO) before treating the samples. Different concentrations (2.5 µM to 25 µM) were studied for the purpose of determining the IC₅₀.

Cell proliferation was evaluated using the MTS [3-(4,5-dimethylthiazol-2-yl)-5-(3-carboxymethoxyphenyl)2-(4-sulfophenyl)-2H-tetrazolium] method, following the instructions for use (CellTiter 96 Aqueous One Solution Cell Proliferation Assay, Promega, USA).

### Cell proliferation method

During the time of the experiment, the cultured cells were detached every 4-5 days by means of trypsinization and the trypan blue method was used for cell viability. The cells were quantified by means of counting method and cultured in 96-well plates. Each experiment included three plates termed T₀, T₁ and T₂.

To contained wells without cells (0 cells/0.1 ml) termed white wells and wells that contained cells (10⁴ cells/0.1 ml) were termed control wells. Both T₁ and T₂ included white wells (0 cells/0.1 ml), control wells (10⁴ cells/0.1 ml) and control wells treated with L-733,060.

In T₀, 20 µl of MTS were immediately added to the wells and they were read 90 minutes later. T1 and T2 were treated with different concentrations (5 µM to 20 µM) of L-733,060 and were incubated during a period of 30 hours (first cell division) (T₁) and 72 hours (second cell division) (T₂).

To study cell proliferation, 20 µl of MTS reagent were added to each well (T₁, T₂) 90 :minutes before reading the samples with the plate reader (TECAN Spectra Classic) at 492 nm. The amount of MTS reagent was measured by optical density, being directly proportional to the number of live cells. Each plate (white, control, and control treated with different concentrations of L-733,060) was done in triplicate. The experiment was repeated on three different occasions. The concentration to inhibit fifty percent of the cells (IC₅₀) with L-733,060 was calculated with a curve suited to the parameters.

### Statistical analysis

The data obtained was evaluated using the student's t test, with a level of significance of P< 0.05.

### Results

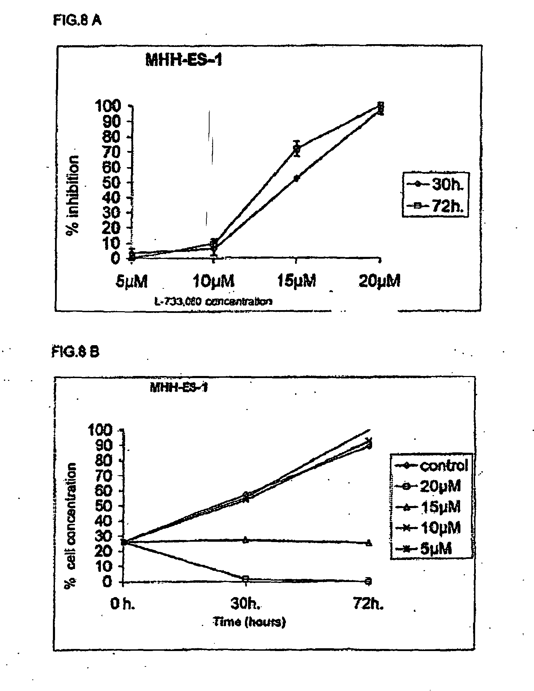

The results shown in Figure 8A represent the variation over time of the concentration of MHH-ES-1 cells at increasing concentrations of L-733,060.

Figure 8B shows cell growth inhibition pf MHH-ES-1 (at 30 and 72 hours) after the addition of increasing concentrations of L-733,060 (5, 10, 15, 20 µM) and the percentage of inhibition for the first and second time of division of the incubation. The discontinuous lines represent the IC₅₀ for 30 and 72 hours. The points on the graph represent the mean value ± standard deviation.

### Example 10: Cell line related to human osteosarcoma

Human osteosarcoma cell line MG-63 (ICLC) was used.

This cell line was maintained in MEN culture medium supplemented with 10% fetal bovine serum according to the cell culture conditions of the ATCC.

The cell line was cultured in 75 ml flasks (Falcon, Germany). The medium was refreshed every two days and the cells were treated with trypsin (0.05% and 0.02% EDTA without Ca²+ and Mg²+) every six days. The cells were incubated at 37ºC under humidification (95% air/5% CO2).

### Treatment with the NK1 receptor antagonist

The solutions of the NK1 receptor antagonist (2S,3S)3-([3,5-Bis(trifluoromethyl)phenyl]methoxy)-2-phenylpiperidine (L-733,060) (Sigma-Aldrich, U.K.) were dissolved in distilled water containing 0.2% dimethyl sulfoxide (DMSO) before treating the samples. Different concentrations (2.5 µM to 20 µM) were studied for the purpose of determining the IC₅₀.

Cell proliferation was evaluated using the MTS [3-(4,5-dimethylthiazol-2-yl)-5-(3-carboxymethoxyphenyl)2-(4-sulfophenyl)-2H-tetrazolim] method, following the instructions for use (CellTiter 96 Aqueous One Solution Cell Proliferation Assay, Promega, USA).

### Cell proliferation method

During the time of the experiment, the cultured cells were detached every 4-5 days by means of trypsinization and the trypan blue method was used for cell viability. The cells were quantified by means of a counting method and cultured in 96-well plates. Each experiment included three plates termed T₀, T₁ and T₂.

T₀ contained wells without cells (0 cells/0.1 ml) termed white wells and wells that contained cells (10⁴ cells/0.1 ml) were termed control wells. Both T1 and T₂ included white wells (0 cells/0.1 ml), control wells (10⁴ cells/0.1 ml) and control wells treated with L-733,060.

In To, 20 µl of MTS were immediately added to the wells and they were read 90 minutes later. T₁ and T₂ were treated with different concentrations (2.5 µM to 25 µM) of L-733,060 and were incubated during a period of 30 hours (first cell division) (T₁) and 72 hours (second cell division)(T₂).

To study cell proliferation, 20 µl of MTS reagent were added to each well (T₁, T₂) 90 minutes before reading the samples with the plate reader (TECAN Spectra Classic) at 492 nm. The amount of MTS reagent was measured by optical density, being directly proportional to the number of live cells. Each plate (white, control, and control treated with different concentrations of L-733,060) was done in triplicate. The experiment was repeated on three different occasions. The concentration to inhibit fifty percent of the cells (IC₅₀) with L-733,060 was calculated with a curve suited to the parameters.

### Statistical analysis

The data obtained was evaluated using the student's t test, with a level of significance of P< 0.05.

### Results

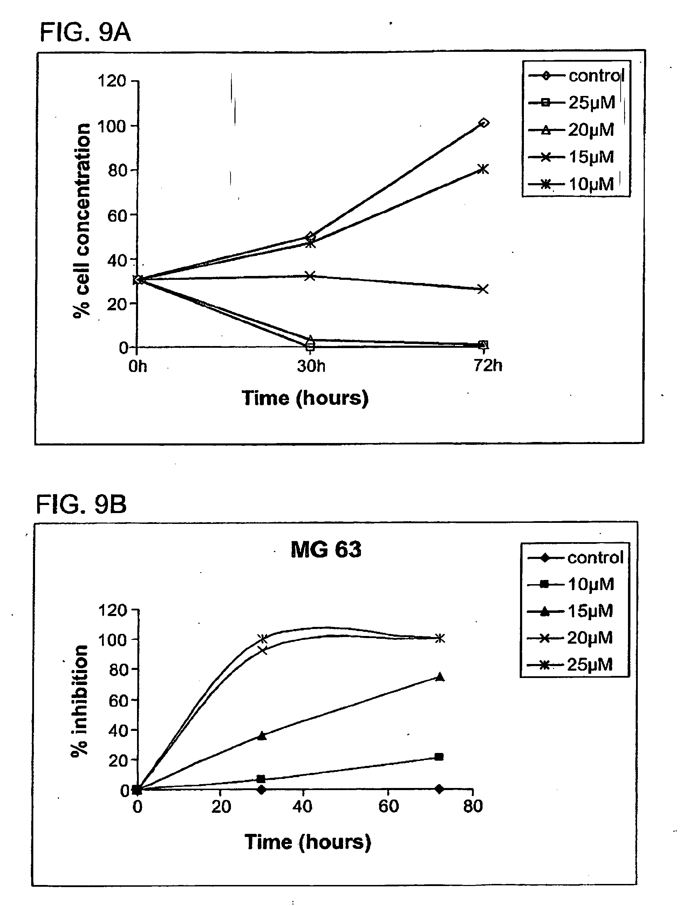

The results shown in Figure 9A represent the variation over time of the concentration of cells at increasing concentrations of L-733,060.

Figure 9B shows cell growth inhibition of MG-63 (at 30 and 72 hours) after the addition of increasing concentrations of L-733,060 (2.5, 5, 10, 20 and 25 µM) and the percentage of inhibition for the first and second time of division of the incubation. The discontinuous lines represent the IC₅₀ for 30 and 72 hours. The points on the graph represent the mean value ± standard deviation.

### Example 11: Cell lines related to glioma

Human glioma-cell line GAMG (DSMZ) was used.

This cell line was maintained in MEN culture medium supplemented with 10% fetal bovine serum according to the cell culture conditions of the ATCC.

The cell line was cultured in 75 ml flasks (Falcon, germany). The medium was refreshed every two days and the cells were treated with trypsin (0.05% and 0.02% EDTA without Ca²+ and Mg²+), every six days. The cells were incubated at 37ºC under humidification (95% air/5% CO₂).

### Treatment with the NK1 receptor antagonist

The solutions of the NK1 receptor antagonist (2S,3S)3-([3,5-Bis(trifluoromethyl)phenyl]methoxy)-2-phenylpiperidine (L-733,060) (Sigma-Aldrich, U.K.) were dissolved in distilled water containing 0.2% dimethyl sulfoxide (DMSO) before treating the samples. Different concentrations (10 µM to 25 µM) were studied for the purpose of determining the IC₅₀.

Cell proliferation was evaluated using the MTS [3-(4,5-dimethylthiazol-2-yl)-5-(3-carboxymethoxyphenyl)2-(4-sulfophenyl)-2H-tetrazolium] method, following the instructions for use (CellTiter 96 Aqueous One Solution Cell Proliferation Assay, Promega, USA).

### Cell proliferation method

During the time of the experiment, the cultured cells were detached every 4-5 days by means of trypsinization and the trypan blue method was used for cell viability. The cells were quantified by means of a counting method and cultured in 96-well plates. Each experiment included three plates termed To, T₁ and T₂.

To contained wells without cells (0 cell/0.1 ml) termed white wells and wells that contained cells (104 cells/0.1. ml) were termed control wells. Both T₁ and T₂ included white wells (0 cells/0.1 ml), control wells (104 cells/0.1 ml) and control wells treated with L-733,060.

In To, 20 µl of MTS were immediately added to the wells and they were read 90 minutes later. T₁ and T₂ were treated with different concentrations (2.5 µM to 25 µM) of L-733,060 and were incubated during a period of 48 hours (first cell division) (T1) and 96 hours (second cell division) (T2).

To study cell proliferation, 20 µl of MTS reagent were added to each well (T₁, T₂) 90 minutes before reading the samples with the plate reader (TECAN Spectra Classic) at 492 nm. The amount of MTS reagent was measured by optical density, being directly proportional to the number of live cells. Each plate (white, control, and control treated with different concentrations of L-733,060) was done in triplicate. The experiment was repeated on three different occasions. The concentration to inhibit fifty percent of the cells (IC₅₀) with L-733,060 was calculated with a curve suited to the parameters.

### Statistical analysis.

The data obtained was evaluated using the student's t test, with a level of significance of P< 0.05.

### Results

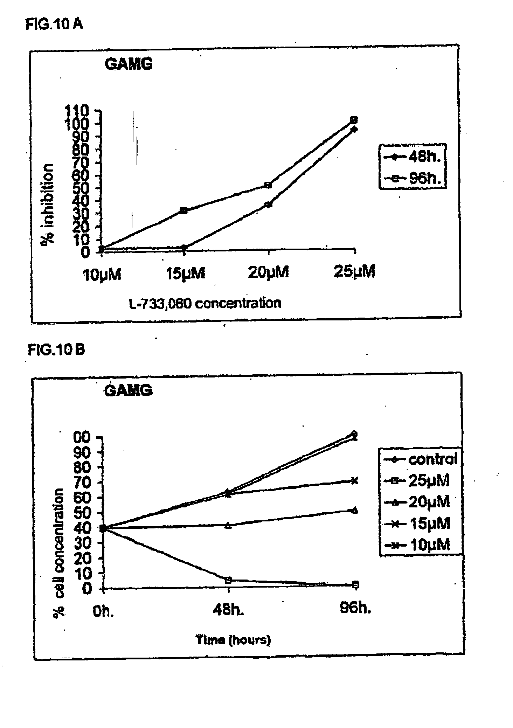

The results shown in Figure 10A represent the variation over time of the concentration of cells at increasing concentrations of L-733,060.

Figure 10B shows cell growth inhibition of GAMG (at 48 and 96 hours) after the addition of increasing concentrations of L-733,060 (10,15, 20 and 25 µM) and the percentage of inhibition for the first and second time of division of the incubation. The discontinuous lines represent the IC₅₀ for 30 and 72 hours. The points on the graph represent the mean value ± standard deviation.

## Claims

1. Composition comprising the non-peptide NK1 receptor and substance P antagonist (2S,3S)3-{[3,5- Bis(trifluoromethyl)phenyl]methoxy}-2-phenylpiperidine, commercially known as L- 733060, for use in the treatment of cancer tumours in mammals.

2. Composition comprising the non-peptide NK1 receptor and substance P antagonist L-733060 for use according to claim 1, wherein the cells of the tumours on which the antagonist acts have a much larger number of NK1 receptors, comprised between 400% and 500% than those existing in non-tumor cells.

3. Composition comprising the non-peptide NK1 receptor and substance P antagonist L-733060 for use according to claims 1 and 2, wherein the tumor cells on which the antagonists act are selected from among:
- primary human invasive malignant melanoma cells
- metastatic human melanoma cells
- melanoma cells located in lymph nodes
- human glioma cells
- human breast cancer cells
- human B cell acute lymphoblastic leukemia cells
- human T cell acute lymphoblastic leukemia cells
- human neuroblastoma cells
- primary human neuroblastoma cells
- human astrocytoma cells
- human Hodgkin's lymphoma cells
- human Ewing's sarcoma cells
- human osteosarcoma cells.

4. Composition comprising the non-peptide NK1 receptor and substance P antagonist L-733060 for use according to claims 1 to 3, wherein the tumor cells related to human melanoma on which the antagonists act belong to the cell lines COLO 858 [ICLC, Interlab Cell Line Collection-CBA-Genoa), MEL HO [DSMZ, Deutsche Sammlung von 35 Mikroorganismen und Zellkulturen] and COLO 679 [DSMZ].

5. Composition comprising the non-peptide NK1 receptor and substance P antagonist L-733060 for use according to claims 1 to 3, wherein the tumor cells related to human glioma and human neuroblastoma on which the antagonists act belong to the cell lines GAMG [DSMZ] and 5 SKN-BE (2) [ICLC].

6. Composition comprising the non-peptide NK1 receptor and substance P antagonist L-733060 for use according to claims 1 to 3, wherein the tumor cells related to lymphoblastic leukemia on which the antagonists act belong to the cell lines of human B cell lymphoblastic leukemia 10 SD1 [DSMZ] and human T cell lymphoblastic leukemia BE-13 [DSMZ].

7. Composition comprising the non-peptide NK1 receptor and substance P antagonist L-733060 for use according to claims 1 to 3, wherein the tumor cells related to human Hodgkin's lymphoma on which the antagonists act belong to the cell line KM-H2 [DSMZ].

8. Composition comprising the non-peptide NK1 receptor and substance P antagonist L-733060 for use according to claims 1 to 3, wherein the tumor cells related to human breast cancer on which the antagonists act belong to the cell line MT-3 [DSMZ].

9. Composition comprising the non-peptide NK1 receptor and substance P antagonist L-733060 for use according to claims 1 to 3, wherein the tumor cells related to human Ewing's sarcoma on which the antagonists act belong to the cell line MHH-ES-1 [DSMZ].

10. Composition comprising the non-peptide NK1 receptor and substance P antagonist L-733060 for use according to claims 1 to 3, wherein the tumor cells related to human osteosarcoma on which the antagonists act belong to the cell line MG-63 [ICLC].

11. Composition comprising the non-peptide NK1 receptor and substance P antagonist L733060 for use, according to claim 1-10, wherein said antagonist is used at concentrations comprised between 5 µM and 50 µM.

## Patentansprüche

1. Zusammensetzung umfassend den nicht-peptid NK1 Rezeptor und Substanz P Antagonisten(2S,3S)3-{[3,5-Bis(trifluoromethyl)phenyl]methoxy}-2-phenylpiperidin, welcher kommerziell als L-733060 bekannt ist, zur Verwendung bei der Behandlung von Krebstumoren in Säugetieren.

2. Zusammensetzung umfassend den nicht-peptid NK1 Rezeptor und Substanz P Antagonisten L-733060 zur Verwendung gemäß Anspruch 1, wobei die Zellen der Tumore, auf welche der Antagonist einwirkt, eine viel größere Anzahl an NK1 Rezeptoren, umfassend zwischen 400% und 500% wie jene, die in Nicht-Tumorzellen vorhanden sind, haben.

3. Zusammensetzung umfassend den nicht-peptid NK1 Rezeptor und Substanz P Antagonisten L-733060 zur Verwendung nach den Ansprüchen 1 und 2, wobei die Tumorzellen, auf welche die Antagonisten einwirken, ausgewählt sind aus:
- Primäre humaninvasive maligne Melanomzellen
- metastatische humane Melanomzellen
- Melanomzellen ansässig in Lymphknoten
- Humane Gliomzellen
- Humane Brustkrebszellen
- Akute lymphoblastische Leukämiezellen humaner B-Zellen
- Akute lymphoblastische Leukämiezellen humaner T-Zellen
- Human Neuroblastomzellen
- Primäre Human Neuroblastomzellen
- Humane Astrocytom-Zellen
- Humane Hodgkin-Lymphomzellen
- Humane Ewing-Sarkomzellen
- Humane Osteosarkomzellen.

4. Zusammensetzung umfassend den nicht-pepid NK1 Rezeptor und Substanz P Antagonisten L-733060 zur Verwendung nach den Ansprüchen 1 bis 3, wobei die Tumorzellen, bezogen auf das humane Melanom, auf welche die Antagonisten einwirken, zu den Zell-Linien COLO 858 [ICLC, Interlab Cell Line Collection-CBA-Genua], MEL HO [DSMZ, Deutsche Sammlung von 35 Mikroorganismen und Zellkulturen] und COLO 679 [DSMZ] gehören.

5. Zusammensetzung umfassend den nicht-pepid NK1 Rezeptor und Substanz P Antagonisten L-733060 zur Verwendung nach den Ansprüchen 1 bis 3, wobei die Tumorzellen, bezogen auf das humane Gliom und das humane Neuroblastom, auf welche die Antagonisten einwirken, zu den Zell-Linien GAMG [DSMZ] und 5 SKN-BE (2) [ICLC] gehören.

6. Zusammensetzung umfassend den nicht-pepid NK1 Rezeptor und Substanz P Antagonisten L-733060 zur Verwendung nach den Ansprüchen 1 bis 3, wobei die Tumorzellen, bezogen auf die lymphoblastische Leukämie, auf welche die Antagonisten einwirken, zu den Zell-Linien der lymphoblastischen Leukämie humaner B-Zellen 10 SD1 [DSMZ] und der lymphoblastischen Leukämie humaner T-Zellen BE-13 [DSMZ] gehören.

7. Zusammensetzung umfassend den nicht-pepid NK1 Rezeptor und Substanz P Antagonisten L-733060 zur Verwendung nach den Ansprüchen 1 bis 3, wobei die Tumorzellen, bezogen auf das humane Hodgkin-Lymphödem, auf welche die Antagonisten einwirken, zu der Zell-Linie KM-H2 [DSMZ] gehören.

8. Zusammensetzung umfassend den nicht-pepid NK1 Rezeptor und Substanz P Antagonisten L-733060 zur Verwendung nach den Ansprüchen 1 bis 3, wobei die Tumorzellen, bezogen auf den humanen Brustkrebs, auf welche die Antagonisten einwirken, zu der Zell-Linie MT-3 [DSMZ] gehören.

9. Zusammensetzung umfassend den nicht-pepid NK1 Rezeptor und Substanz P Antagonisten L-733060 zur Verwendung nach den Ansprüchen 1 bis 3, wobei die Tumorzellen, bezogen auf das Ewing-Sarkom, auf welche die Antagonisten einwirken, zu der Zell-Linie MHH-ES-1 [DSMZ] gehören.

10. Zusammensetzung umfassend den nicht-pepid NK1 Rezeptor und Substanz P Antagonisten L-733060 zur Verwendung nach Ansprüchen 1 bis 3, wobei die Tumorzellen, bezogen auf das humane Osteosarkom, auf welche die Antagonisten einwirken, zu der Zell-Linie MG-63 [ICLC] gehört.

11. Zusammensetzung umfassend den nicht-pepid NK1 Rezeptor und Substanz P Antagonisten L-733060 zur Verwendung nach Anspruch 1 - 10, wobei der Antagonist bei Konzentrationen, umfassend zwischen 5 µM und 50 µM, benutzt wird.

## Revendications

1. Composition comprenant le récepteur non-peptide NK1 et la substance P antagoniste (2S, 3S) 3-{[3,5- Bis(trifluoromethyl) phenyl]methoxi}-2-phenylpipéridine, connue sous le nom commercial L- 733060, destinéee à un usage dans le traitement de tumeurs cancéreuses de mammifères.

2. Composition comprenant le récepteur non-peptide NK1 et la substance P antagoniste L-733060, destinée à l'usage selon la revendication 1, dans laquelle les cellules tumorales sur lesquelles l'antagoniste agit ont un bien plus grand nombre de récepteurs NK1, compris entre 400% et 500%, que ceux existant dans des cellules non-cancéreuses.

3. Composition comprenant le récepteur non-peptide NK1 et la substance P antagoniste L-733060, destinée à l'usage selon les revendications 1 et 2, dans laquelle les cellules tumorales sur lesquelles les antagonistes agissent sont sélectionnées parmi :
- des cellules humaines de mélanomes malignes primaires invasives
- des cellules humaines de mélanome métastatique
- des cellules de mélanomes localisées dans les ganglions lymphatiques ganglionnaires
- des cellules humaines de gliome
- des cellules humaines de cancer du sein
- des cellules humaines B de leucémie lymphoblastique aiguë
- des cellules humaines T de leucémie lymphoblastique aiguë
- des cellules humaines de neuroblastome
- des cellules humaines de neuroblastome primaires
- des cellules humaines d'astrocytome
- des cellules humaines de lymphome de Hodgkin
- des cellules humaines de sarcome de Edwing
- les cellules humaines d'ostéosarcome

4. Composition comprenant le récepteur non-peptide NK1 et la substance P antagoniste L-733060, destinée à l'usage selon les revendications 1 à 3, dans laquelle les cellules tumorales liées à un mélanome humain sur lesquelles les antagonistes agissent appartiennent aux lignées cellulaires COLO 858 [ICLC, Interlab Cell Line Collection-CBA-Genova), MEL HO [DSMZ, Deutsche Sammlung von 35 Mikroorganismen und Zellkulturen] et COLO 679 [DSMZ].

5. Composition comprenant le récepteur non-peptide NK1 et la substance P antagoniste L-733060, destinée à l'usage selon les revendications 1 à 3, dans laquelle les cellules tumorales liées à un gliome humain et neuroblastome humain sur lesquelles les antagonistes agissent appartiennent aux lignées cellulaires GAMG [DSMZ] et 5 SKN-BE (2) [ICLC].

6. Composition comprenant le récepteur non-peptide NK1 et la substance P antagoniste L-733060, destinée à l'usage selon les revendications 1 à 3, dans laquelle les cellules tumorales liées à une leucémie lymphoblastique sur lesquelles les antagonistes agissent appartiennent aux lignées cellulaires de cellules humaines B de leucémie lymphoblastique 10 SD1 [DSMZ] et de cellules humaines T de leucémie lymphoblastique BE-13 [DSMZ].

7. Composition comprenant le récepteur non-peptide NK1 et la substance P antagoniste L-733060, destinée à l'usage selon les revendications 1 à 3, dans laquelle les cellules tumorales liées à un lymphome de Hodgkin sur lesquelles les antagonistes agissent appartiennent à la lignée cellulaire KM-H2 [DSMZ].

8. Composition comprenant le récepteur non-peptide NK1 et la substance P antagoniste L-733060, destinée à l'usage selon les revendications 1 à 3, dans laquelle les cellules tumorales liées à un cancer du sein humain sur lesquelles les antagonistes agissent appartiennent à la lignée cellulaire MT-3 [DSMZ].

9. Composition comprenant le récepteur non-peptide NK1 et la substance P antagoniste L-733060, destinée à l'usage selon les revendications 1 à 3, dans laquelle les cellules tumorales liées à un de sarcome de Edwing sur lesquelles les antagonistes agissent appartiennent à la lignée cellulaire MHH-ES-1 [DSMZ].

10. Composition comprenant le récepteur non-peptide NK1 et la substance P antagoniste L-733060, destinée à l'usage selon les revendications 1 à 3, dans laquelle les cellules tumorales liées à un ostéosarcome humaine sur lesquelles les antagonistes agissent appartiennent à la lignée cellulaire MG-63 [ICLC].

11. Composition comprenant le récepteur non-peptide NK1 et la substance P antagoniste L-733060, destinée à l'usage selon les revendications 1 à 10, dans laquelle ledit antagoniste L-733060 est utilisé dans des concentrations comprises entre 5µM et 50 µM.
